# EUROPEAN PATENT SPECIFICATION

(11) **EP 1 713 396 B1**
(45) Date of publication and mention of the grant of the patent: **27.07.2011**
(21) Application number: 05702848.2
(22) Date of filing: 31.01.2005
(51) Int. Cl.: A61B 8/00

(54) **DIAGNOSTIC ULTRASOUND SYSTEM WITH ARTICULATING FLAT PANEL DISPLAY**
DIAGNOSTISCHES ULTRASCHALLSYSTEM MIT GELENKIGER FLACHBILDSCHIRMANZEIGE
SYSTEME ULTRASONORE DE DIAGNOSTIC PRESENTANT UN ECRAN PLAT ARTICULE

(30) Priority: 06.02.2004 US 542793 P
(43) Date of publication of application: 25.10.2006
(73) Proprietor: Koninklijke Philips Electronics N.V., 5621 BA Eindhoven (NL)
(72) Inventor: MURKOWSKI, John, Bothell, WA 98041-3003 (US); MESAROS, Robert, Bothell, WA 98041-3003 (US); AZZANO, Larry, Bothell, WA 98041-3003 (US)
(74) Representative: Roche, Denis
(86) International application number: PCT/IB2005/050405
(87) International publication number: WO 2005/074806

(56) References cited:
- US-A- 5 826 846
- US-A- 5 924 988
- US-A- 2003 220 564
- US-A1- 2003 220 565
- US-B1- 6 669 639
- PATENT ABSTRACTS OF JAPAN vol. 017, no. 285 (C-1066), 2 June 1993 (1993-06-02) & JP 05 015529 A (MATSUSHITA ELECTRIC IND CO LTD), 26 January 1993 (1993-01-26)

## Description

This invention relates to medical diagnostic imaging systems and, in particular, to ultrasonic diagnostic imaging systems with flat panel displays that can be easily positioned for viewing by a user.

Ultrasound systems are now being designed to be more ergonomically comfortable for the user to operate. Often, the ultrasound system is wheeled to the patient's bedside for imaging. The sonographer must then be able to hold the probe in contact with the patient while operating the ultrasound system controls and viewing the images produced on the system image display. To enable the sonographer to assume a comfortable position while doing this, one which is primarily focused on the patient, it is desirable for the system controls and display to be movable to a comfortable operating and viewing position. For instance, US pat. app. 2003,0220564A1, describes a Cart-borne ultrasound system improving the ergonomics of an articulated control panel. In this effect, the control panel is mounted to the cart of the system via a 4-bar linkage articulation mechanism.

US pat. 5,924,988 describes an ultrasound system comprising a flat panel display secured to a structure. According to this document the structure enables the operator to position the flat panel display with superior ergonomics.

JP pat. app. 03170967 discloses an ultrasound system with an articulation mechanism comprising a bar-linkage. The mechanism is mounted between a CRT monitor and a rotary base of the system such that fatigue of the operator can be reduced according to this document.

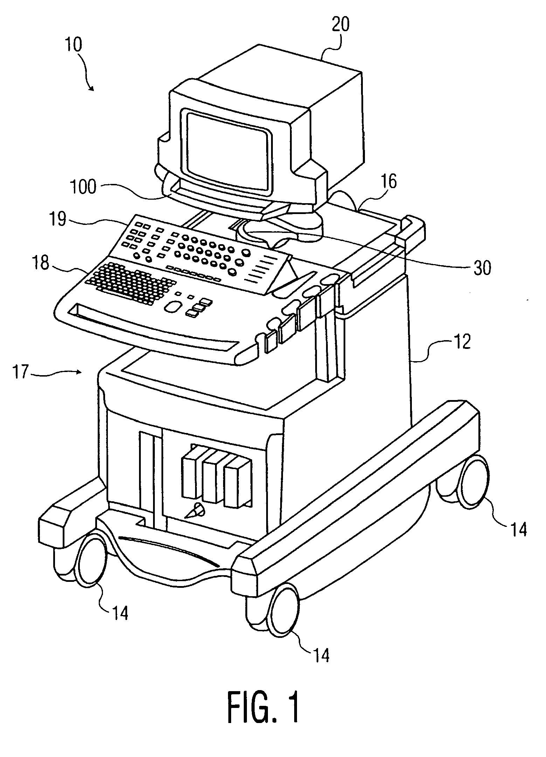

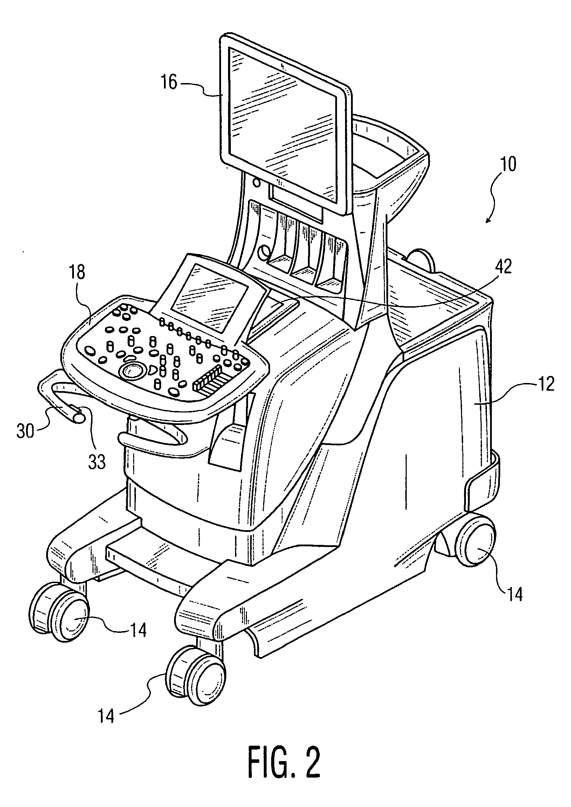

As another example US pat. 6,669,639 (Miller et al.) describes the ultrasound system shown in FIGURE 1. The display monitor 20 of this system is mounted on a 2-arm articulating mount 30 on the upper surface of the system cart 12, which enables the monitor to be moved from side to side of the ultrasound system cart and to be rotated toward the sonographer or patient for easy viewing. US pat. [application serial number 10/155,459, entitled "DIAGNOSTIC ULTRASOUND SYSTEM CART WITH LATERALLY ARTICULATING CONTROL PANEL,"] describes the ultrasound system shown in FIGURE 2, which uses a flat panel display 16 mounted at a nominal position above the system control panel 18. The control panel 18 of this system can move from one side of the system cart to the other and can rotate or swivel toward the sonographer for comfortable bedside operation. It would be desirable for the flat panel display 16 to be similarly movable to a comfortable viewing position. An optimal design would enable the display 16 to be positioned over a wide range of lateral viewing positions and heights, and to be easy for the sonographer to reposition with one hand.

In accordance with the principles of the present invention an ultrasound system is described with a flat panel display that articulates to be viewed over a wide range of viewing positions. The articulation is provided by a 2-arm articulation system with a counter-weight assisted 4-bar linkage. The counter-weight assist and the 4-bar linkage require very little effort to reposition the display to a comfortable viewing position. The flat panel display has a peripheral gripping surface which enables the display to be held and repositioned with one hand so that the display can be easily adjusted to be viewable by esther the sonographer or the patient.

In the drawings:
FIGURE 1 illustrates a cart-borne ultrasound system with an articulating monitor.
FIGURE 2 illustrates a cart-borne ultrasound system with a flat panel display and an articulating control panel.
FIGURE 3 illustrates an articulating flat panel display for an ultrasound system constructed in accordance with the principles of the present invention.
FIGURE 4 are forward and rearward perspective views of an articulating flat panel display of the present invention.
FIGURE 5 illustrates a flat panel display with a peripheral gripping surface for articulation.
FIGURE 6 is a partially cutaway view of the flat panel display of FIGURE 5, showing the back gripping surface.
FIGURES 7a and 7b are cross-sectional views of the flat panel display of FIGURE 5.
FIGURE 8a, 8b and 8c illustrate an articulating flat panel display of the present invention in the raised, nominal, and stowed positions.
FIGURE 9 illustrates the range of articulation of an articulating flat panel display of the present invention in a vertical plane.
FIGURES 10a, 10b, and 10c illustrate the lateral range of articulation of an articulating flat panel display of the present invention.
FIGURE 11 is an illustration of a number of laterally articulated positions of an articulating flat panel display of the present invention.
FIGURE 12 illustrates a cart-borne ultrasound system of the present invention with a laterally articulating control panel and an articulating flat panel display.

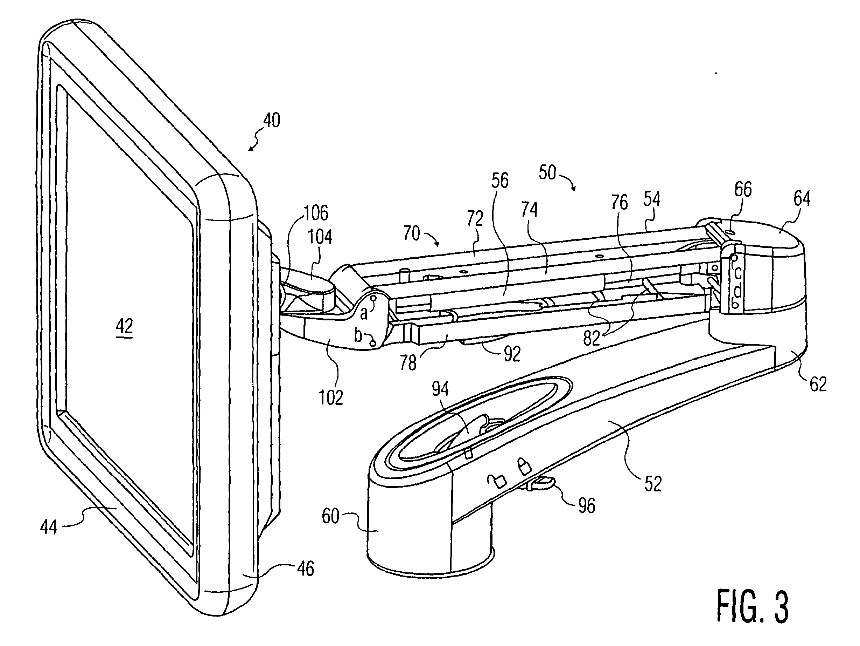

Referring now to FIGURE 3, an articulating flat panel display assembly constructed in accordance with the principles of the present invention is shown.
The flat panel display 40 has a viewing screen 42 which is enclosed in an enclosure having a front bezel 44 which surrounds the edges of the display in the front and a rear enclosure section 46. The front bezel 44 and the periphery of the rear enclosure section 46 include gripping surfaces by which a user can grip the flat panel display to adjust its position. The flat panel display 40 is mounted to the ultrasound system by an articulating arm assembly 50. A lower arm (or main base) 52 has a mounting end 60 which is mounted to the ultrasound system. The lower arm 52 is pivotally mounted to the ultrasound system to pivot about a vertical pivot axis extending through the mounting end 60. The mounting end 60 encloses a circular mounting bracket with a tooth that rides in a circular slot inside the mounting end 60. The circular slot extends only halfway around the pivot axis of the mounting end and thereby restrains the range of pivoting of the mounting end to 180°. It is desirable to prevent continuous unlimited rotation of the lower arm because the cables to the flat panel display extend through the articulating arms. Continuous rotation of the arms would cause these cables to become twisted and ultimately to be damaged.

The lower arm 52 of the articulating arm assembly 50 is angled upward at a fixed angle of approximately 25°. This upward angling of the lower arm 52 provides elevation for the upper arm and flat panel above the upper surface of the ultrasound system. This elevation provides clearance above parts or accessories of the system that may be located above or placed on the upper surface of the system. The elevation also raises the upper arm to a level where it will locate the flat panel display in a nominal, neutrally balanced viewing position when the upper arm is oriented horizontally.

The upper end 62 of the lower arm 52 mates with the elbow 64 of the upper arm 54. The elbow 64 and upper end 62 are pivotally connected so that the elbow joint will pivot about a second vertical axis. The upper end 62 of the lower arm includes a pin which rides in a groove in the inner sleeve of the elbow. The pin and groove of this pivoting connection function in the same manner as the tooth and circular slot of the mounting end 60, allowing the two arms to rotate through a limited arc of the circular 180° groove. The elbow rotation is thus prevented from unlimited rotation which could damage the flat panel cables.

The upper arm 54 includes a 4-bar linkage 70.
The four bars 72, 74, 76 and 78 of the linkage 70 are pivotally connected by pivot pins a and b a the forward end of the arm 54 and by pivot pins c and d at the elbow end of the arm. The 4-bar linkage 70 enables the flat panel display to be raised and lowered with respect to the elbow 64. When the upper arm 54 is viewed from the side, the ends of the pivot pins a, b, c, and d will always form a parallelogram as the linkage is articulated up and down. The two upper bars 72 and 74 of the linkage 70 are in this embodiment formed by the two sides of a U-shaped steel plate. The use of the steel plate for the two upper bars provide strength and rigidity between the two bars. The two lower bars 76 and 78 in this embodiment are formed by separate bars which are connected by ribs 82 for strength and rigidity between the lower bars.

Contained within the four bars of the 4-bar linkage 70, in addition to the cabling to the flat panel display, is a pneumatic piston (or shock) 56. The compressive force of the piston 56 provides a counter force to the weight of the flat panel display. The pneumatic piston 56 is pivotally connected to a tilt/swivel base 102 at one end of the upper arm 54, and is also pivotally connected to the elbow 64 at the other end of the upper arm 54. The pivot connection at the elbow 64 is mounted on a threaded shaft vertically positioned in the elbow 64.
A hole 66 in the elbow 64 provides access to a hex-shaped head of the threaded shaft. As the threaded shaft is turned the pivot connection of the pneumatic piston will move up or down in relation to the c and d pivots of the 4-bar linkage. This repositioning of the elbow end of the piston will increase or decrease the tension or stiffness of the force provided by the piston. When the piston force is made stiffer, the user will have to use more force to move the flat panel display down and less force to move the display up. When the piston force is made less stiff, the user will be able to use less force to move the display down and more force to move the display up.

The tilt/swivel base 102 is pivotally connected to a tilt/swivel bracket 104. This connection permits the bracket 104 to rotate about a vertical axis passing through the base 102 and bracket 104, enabling the flat panel display to be turned from side to side without repositioning the articulating arm assembly 50. The tilt/swivel bracket 104 is pivotally mounted to the rear enclosure 46 of the flat panel display by a pivot connection 106 which pivots around a horizontal pivot axis. This pivot axis permits the flat panel display to be tilted to face upward or downward without moving the articulating arm assembly.

Extending downward from the bottom of the upper articulating arm 54 is a catch plate 92. The catch plate 92 will engage a spring-loaded lock plate 94 in the lower arm 52 when the two arms are brought together. The catch plate 92 will contact a spring-loaded ball which is partially visible to the right of the lock plate 94 when the lock is open, which causes the lock plate 94 to spring to the right and retain the catch plate 92. A lock release 96 on the bottom of the lower arm will then move to the locked position. The two arms will remain locked together until the lock release 96 is moved to the unlock position, which moves the lock plate 94 to the left and releases the engaged catch plate 92. The two arms are locked together to secure the articulating display and prevent articulation when the ultrasound system is being moved or transported.

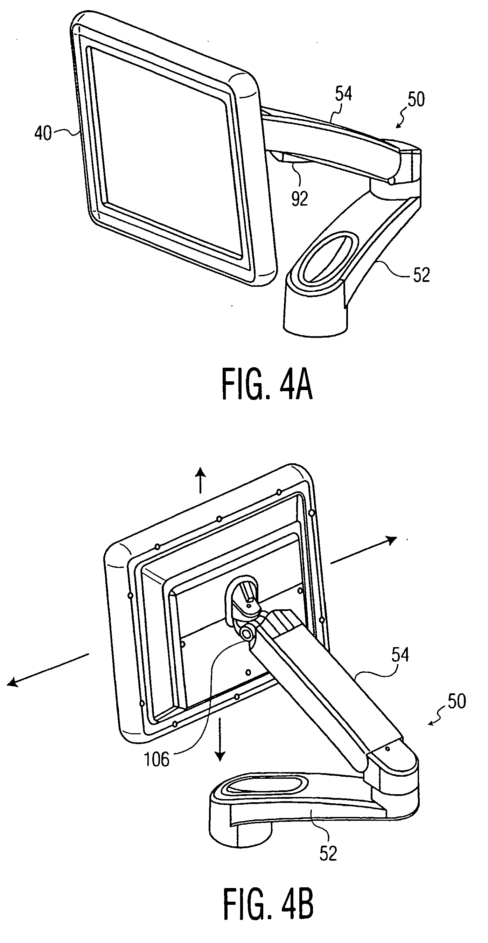

FIGURE 4a is a perspective view of the articulating flat panel display when the 4-bar linkage upper arm 54 is raised above the horizontal to elevate the display to a higher viewing position. FIGURE 4b is a view of the same positioning of the articulating display from the rear of the flat panel display. As the arrows in FIGURE 4b indicate, the flat panel display can be repositioned horizontally by operation of the vertical axis pivots of the assembly, and the flat panel display can be moved vertically by movement of the bars of the 4-bar linkage 70.

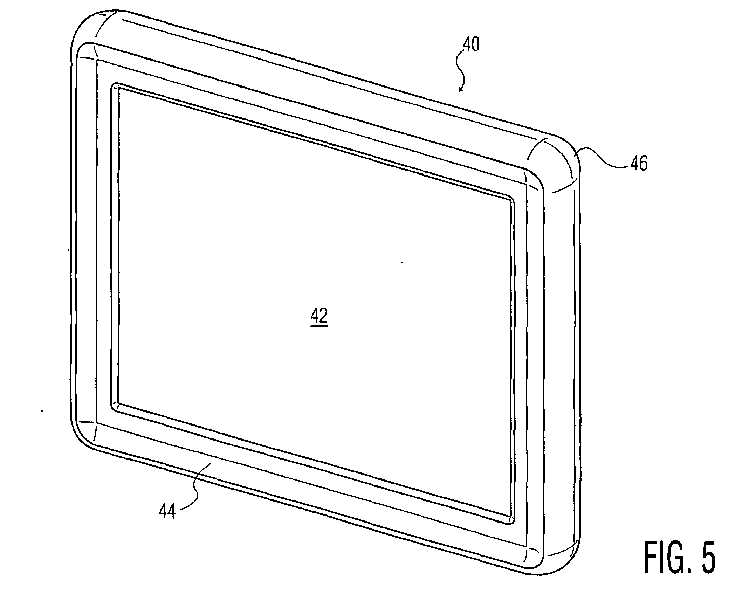

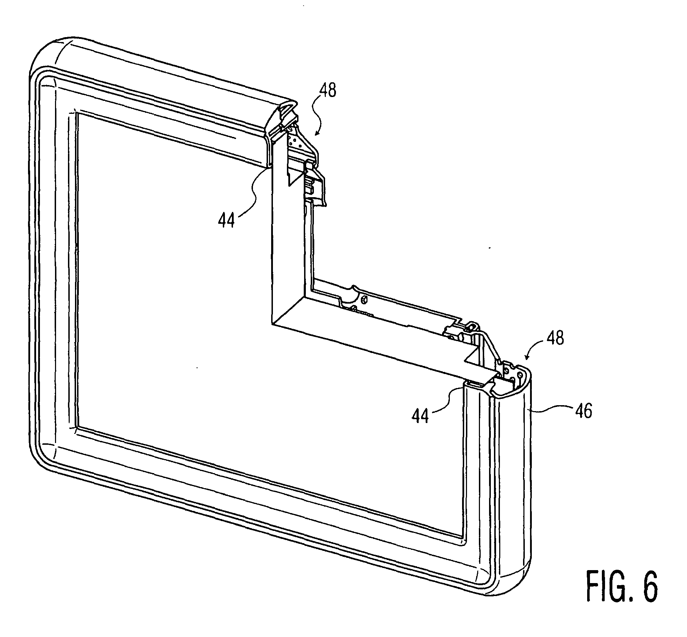

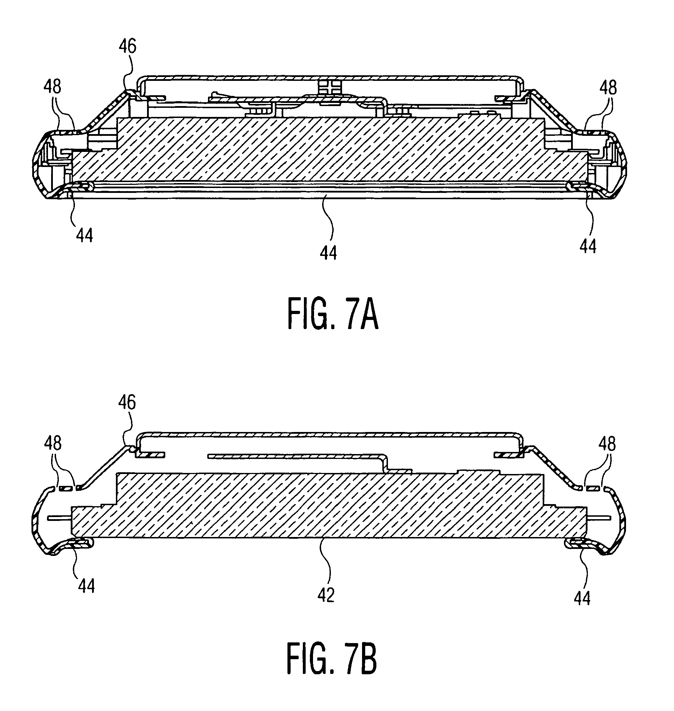

FIGURE 5 shows a front view of a flat panel display embodying a further aspect of the present invention. FIGURE 1 shows the monitor 20 of the system 10 there shown with a handle 100 on the front of the monitor. The monitor can be repositioned by grasping the handle to move the monitor. A flat panel display, lacking the weight of the glass of a display monitor, does not require a repositioning means as robust as a handle. In the embodiment of FIGURE 5 the bezel 44 around the front of the display screen 42 has a surface designed to be gripped by the user's thumb when repositioning the flat panel display. This gripping surface can be provided by forming the front bezel 44 of a silicon or rubberlike material. In a constructed embodiment the gripping surface is formed by coating a bezel formed of hard plastic with a thermoplastic elastomeric coating such as a Sanopreen overmold or Soft-touch spray coating available from Also Corp. of Vermon, California, USA. FIGURE 6 is a partially cutaway perspective view of the flat panel display 40 which better illustrates the curvature of the bezel 44 which provides a contoured surface that can be gripped firmly with the user's thumb without slipping. Also visible in the cutaway view is the periphery of the rear enclosure section 46 which also contains a gripping surface for the user's fingers. This gripping surface is provided by the featuring of the surface of the periphery of the section 46. In this case the texturing is provided by perforations 48 through the section 46. These perforations not only provide a good gripping surface on the back periphery of the flat panel display, but also provide ventilation of the flat panel enclosure. FIGURE 7a is a cross-sectional view of the flat panel display which shows the contour of the bezel 44 on the front and the perforations 48 around the rear periphery of the display. FIGURE 7b is a simplification of FIGURE 7a which clearly shows the contoured gripping surface 44 on the front of the flat panel display and the perforated gripping surface 48 on the rear enclosure section 46. One skilled in the art will appreciate that texturing other than perforations may be employed, such as grooves, protrusions, or a roughened surface for gripping.

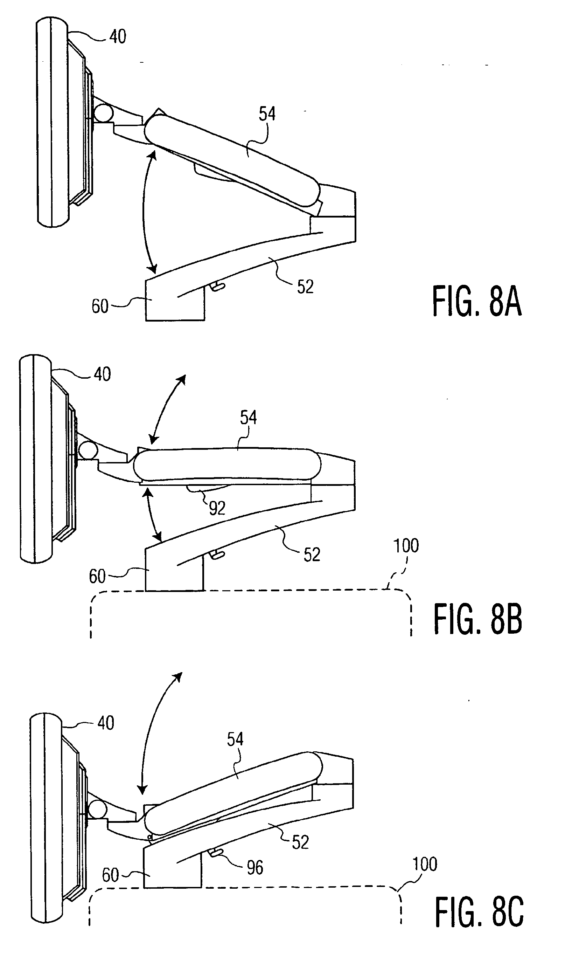

FIGURES 8a-8c illustrate several vertical articulation positions of an embodiment of the present invention. In a constructed embodiment the surface 100 of the ultrasound system on which the articulating flat panel display is mounted is at a height relative to the control panel such that a nominal display position is provided when the upper arm z1 is horizontally oriented as shown in FIGURE 8b. The threaded adjustment of the piston 56 is set so that the piston force will offset the weight of the upper arm 54 and the flat panel 40 when the upper arm 54 is in this horizontal position. The upper arm 54 can then be raised and lowered from this nominal position as indicated by the arrows in the drawing, with the piston force contiguously providing a balancing counter-force. This is due to the use of the 4-bar linkage 70 and the pneumatic piston (shock) 56 for the upper arm 54. If the 4-bar linkage were located in the lower arm 52, for instance, there would be a greater disparity between the force required to raise the display and the force required to lower the display. By locating the 4-bar linkage and piston in the upper arm 54, these forces can be more uniformly balanced.

FIGURE 8c shows the articulating arm assembly when the flat panel display is stowed for travel. The upper arm 54 is lowered as indicated by the arrow until the catch plate 92 engages the lock plate 94, which causes the lock to engage and retain the two arms in the illustrated stowed position.

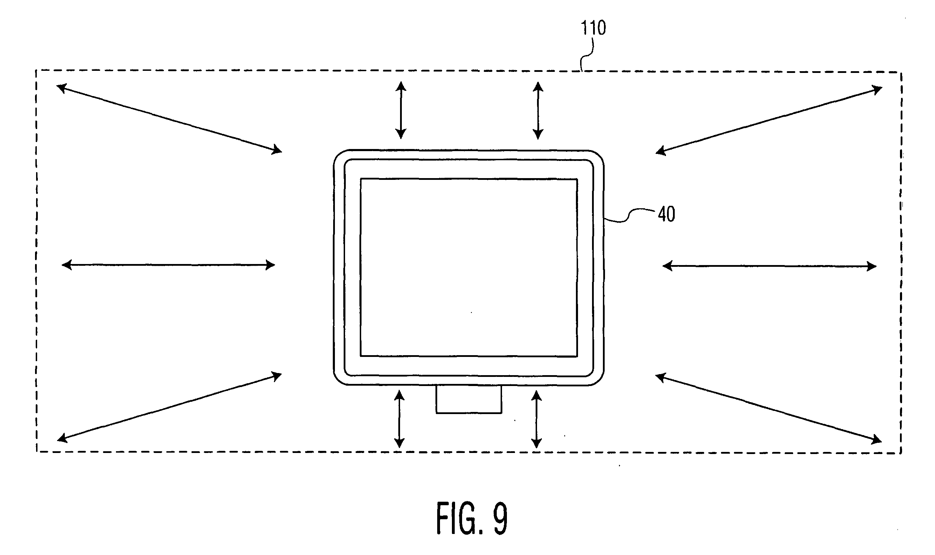

FIGURE 9 illustrates the range 110 of locations in a vertical plane which the flat panel display 40 can assume by use of the articulating arm assembly of FIGURE 3. As the arrows indicate the display 40 can move vertically up or down by reason.of the articulation of the 4-bar linkage 70 of the upper arm 54. When the articulating arms are pivoted about their vertical pivot axes, the flat panel display can be repositioned from side to side as indicated by the arrows on either side of the display 40.

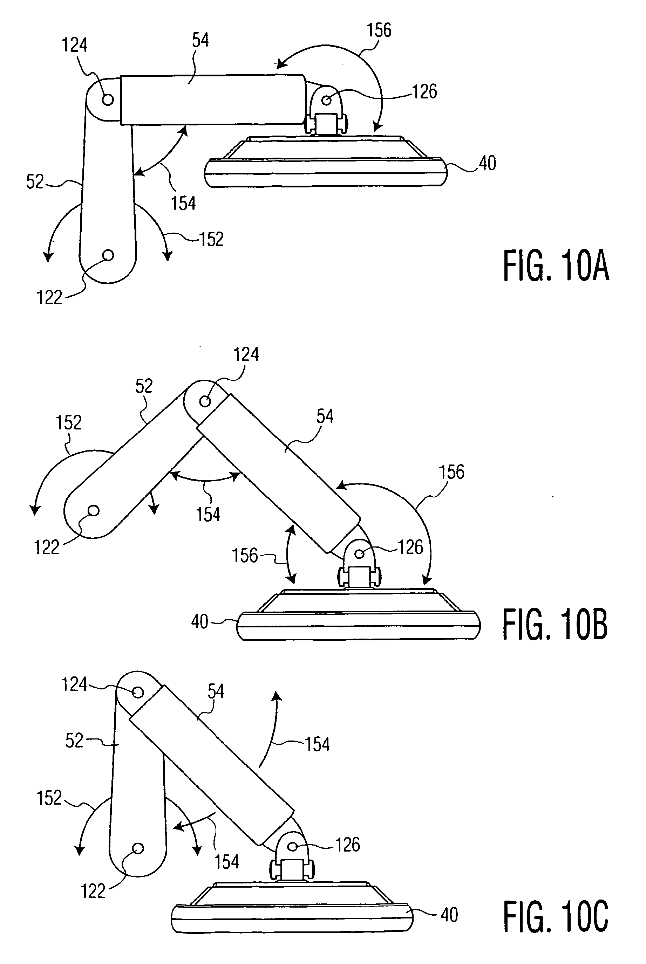

FIGURES 10a-10c illustrate the lateral articulation provided by the vertical pivot axes of the embodiment of FIGURE 3. The pivot axis 122 passes through the mounting end 60 of the lower arm 52, the pivot axis 124 passes through the elbow 64 of the articulating arms, and the pivot axis 126 passes through the tilt/swivel base 102 at the back of the flat panel display 40. As explained previously, the pivoting of the lower arm 52 about the axis 122 is constrained to 180° as shown by arrows 152. The pivoting of the upper arm 54 about the axis 124 is also constrained to 180° as shown by the arrows 154. The flat panel display 40 can pivot over the full range about the end of the upper arm 54 as shown by the arrows 156. In FIGURE 10a the lower arm 52 is in its "home" position in which it extends to the rear of the ultrasound system and the upper arm 54 has been pivoted 90° to the right. The flat panel display has been pivoted about axis 126 to face forward. This, and the orientations of the other two drawings on this sheet, show how the flat panel display may be positioned when the sonographer is diagnosing a patient located on the right side of the ultrasound system. In FIGURE 10b the flat panel display has been brought forward and slightly more to the right by pivoting the lower arm 52 about axis 122 and adjusting the flat panel display about axis 126. In FIGURE 10c the flat panel display is moved toward the center of the ultrasound system by movement about all three axes from the position shown in FIGURE 10b.

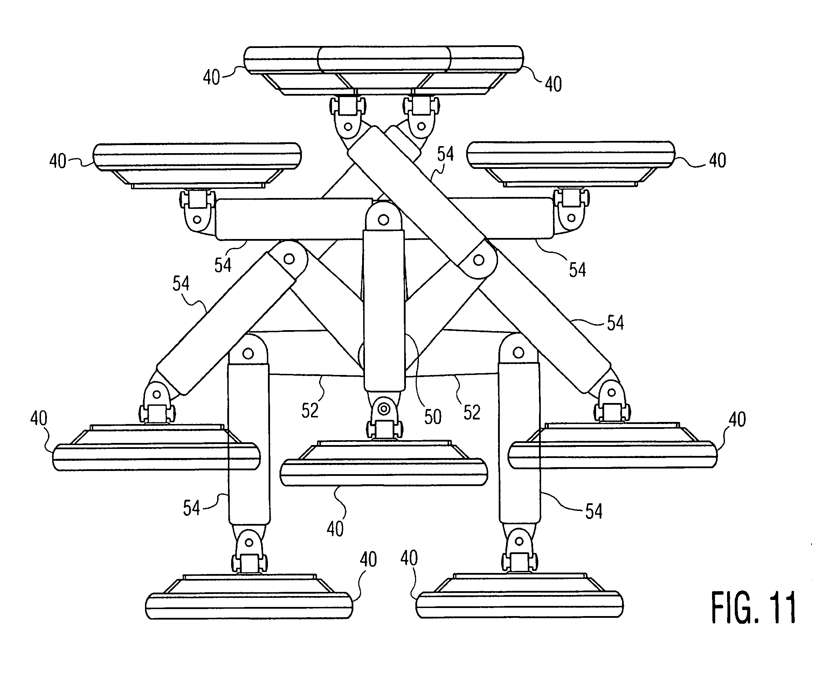

FIGURE 11 shows an embodiment of the present invention with a wide range of lateral display positions. Shown in bold in the center of FIGURE 11 is a flat panel display 40 and articulating arm assembly 50 with their arms in the nominal home position. The display screen is facing to the front of the ultrasound system, the lower articulating arm extends toward the rear of the ultrasound system and the upper articulating arm extends from the elbow with the lower arm at the rear to the front of the ultrasound system. Shown in shadow are many of the positions around the mounting point of the articulating arm assembly in which the flat panel display can be located. As the positions at the top of the drawing illustrate, the flat panel display can even be moved to face to the rear of the ultrasound system, should that become desirable.

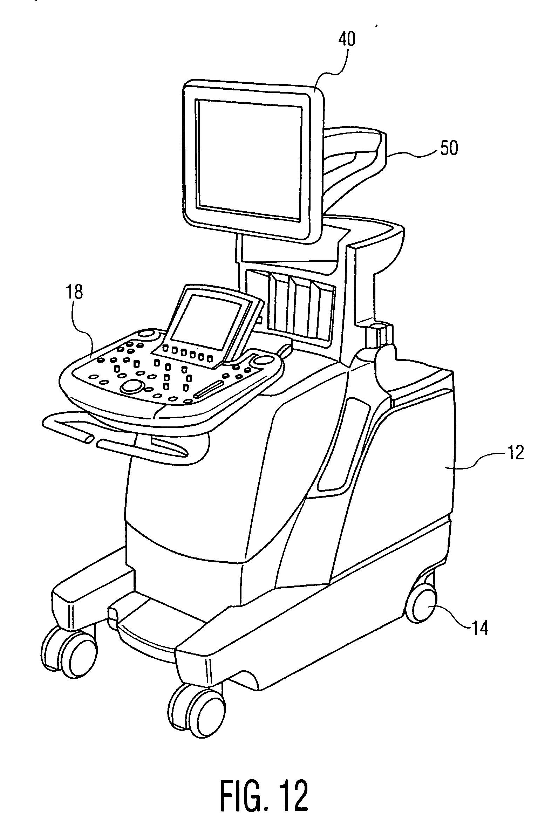

FIGURE 12 illustrates an embodiment of the present invention in which the ultrasound system has both an articulating flat panel display 40 and an articulating control panel 18, both of which can be repositioned laterally with respect to the main body 12 of the ultrasound system cart. The flat panel display may be articulated as described in any of the articulating arm embodiments described above, or by means of other articulating mechanisms. The control panel 18 may be articulated laterally such as described in US-A1 2003/0220565 . With the ability to articulate both the flat panel display 40 and the control panel, the sonographer can configure the ultrasound system to scan a patient in the utmost of comfort and convenience.

## Claims

1. An ultrasonic diagnostic imaging system including a main body (12) housing imaging electronics and a control panel (18) coupled to the imaging electronics comprising:
a flat panel display (40) electrically coupled to the imaging electronics;
an articulating arm assembly (50) to which the flat panel display (40) is connected for adjusting the viewing position of the flat panel display, the articulating arm assembly (50) including a first lower arm (52) movably mounted to the main body and a second upper arm (54) movably connected to the first lower arm (52) and to the flat panel display (40), **characterised in that** at least one of the arms (52,54) includes a 4-bar linkage (70), and
an inter-locking mechanism (92, 94), located on the first and second arms (52, 54), which acts to lock the two arms (52, 54) together, thereby restricting relative motion between the two arms.

2. The ultrasonic diagnostic imaging system of Claim 1, further comprising a wheeled (14) cart on which the main body (12) is mounted.

3. The ultrasonic diagnostic imaging system of Claim 1, wherein the second upper arm (54) includes the 4-bar linkage (70).

4. The ultrasonic diagnostic imaging system of Claim 3, wherein the 4-bar linkage (70) includes first and second pivot axes (c, d) located at an end of the second upper arm (54) which is connected to the first lower arm (52), and third and fourth pivot axes (a, b) located at an end of the second upper arm (54) which is connected to the flat panel display (40).

5. The ultrasonic diagnostic imaging system of Claim 1, wherein the locking mechanism (92, 99) further comprises a user-operable lock release (96) which is operated to cause the locking of the two arms (52, 54) to be released.

6. The ultrasonic diagnostic imaging system of Claim 1, wherein the articulating arm assembly (50) further includes a first vertical pivot axis (122) located at an end of the first lower arm (52) which is movably mounted to the main body, and a second vertical pivot axis (124) located at an end of the first lower arm (52) which is connected to the second upper arm (54).

7. The ultrasonic diagnostic imaging system of Claim 6, wherein the articulating arm assembly (50) further includes a third vertical pivot axis (126) located at an end of the second upper arm (54) which is connected to the flat panel display (40), and a horizontal pivot axis located at the end of the second upper arm (54) which is connected to the flat panel display (40).

8. The ultrasonic diagnostic imaging system of Claim 6, wherein the arc of travel of the first lower arm (52) about the first vertical pivot axis (122) is constrained to be less than 360°, and wherein the arc of travel of the second upper arm (54) about the second vertical pivot axis (124) is constrained to be less than 360°.

9. The ultrasonic diagnostic imaging system of Claim 1, wherein the second upper arm (54) further includes:
a pneumatic piston (56) which acts to provide a force which at least partially offsets the weight of the flat panel display (40).

10. The ultrasonic diagnostic imaging system of Claim 9, further comprising an adjustment mechanism, coupled to the pneumatic piston (56), which is operable to adjust the force provided by the pneumatic piston.

11. The ultrasonic diagnostic imaging system of Claim 10, wherein the pneumatic piston (56) is adjustable to provide a balancing counter-force when the second upper arm (54) is oriented in a horizontal orientation.

12. The ultrasonic diagnostic imaging system of Claim 1, wherein the first lower arm (52) exhibits a fixed upward inclination from an end which is connected to the main body (12) to a second end which is elevated above the connection to the main body.

13. The ultrasonic diagnostic imaging system of Claim 3, wherein the 4-bar linkage (70) includes first and second upper bars (72, 74) coupled between the first and third pivot axes (a, c) and third and fourth lower bars (76, 78) coupled between the second and fourth pivot axes (b, d),
wherein the first bar (72) is rigidly connected to the second bar (74) and the third bar (76) is rigidly connected to the fourth bar (78).

## Patentansprüche

1. Diagnostisches Ultraschall-Bildgebungssystem mit einem Hauptkörper (12), in dem Bildgebungselektronik und ein mit der Bildgebungselektronik gekoppeltes Bedienfeld (18) untergebracht sind, mit:
einer Flachbildschirmanzeige (40), die elektronisch mit der Bildgebungselektronik gekoppelt ist;
einer gelenkigen Armbaugruppe (50), mit der die Flachbildschirmanzeige (40) verbunden ist, um die Sichtposition der Flachbildschirmanzeige zu justieren, wobei die gelenkige Armbaugruppe (50) einen ersten unteren Arm (52), der beweglich an dem Hauptkörper angebracht ist, und einen zweiten oberen Arm (54), der beweglich mit dem ersten unteren Arm (52) und der Flachbildschirmanzeige (40) verbunden ist, umfasst, **dadurch gekennzeichnet, dass** mindestens einer der Arme (52, 54) ein 4-Stangen-Gestänge (70) umfasst, und
einen Verriegelungsmechanismus (92, 94), der sich an dem ersten und dem zweiten Arm (52, 54) befindet und dazu dient, die beiden Arme (52, 54) miteinander zu verriegeln und **dadurch** die relative Bewegung zwischen den beiden Armen zu begrenzen.

2. Diagnostisches Ultraschall-Bildgebungssystem nach Anspruch 1, weiterhin mit einem mit Rädern (14) ausgestatteten Rollwagen, auf dem der Hauptkörper (12) montiert ist.

3. Diagnostisches Ultraschall-Bildgebungssystem nach Anspruch 1, wobei der zweite obere Arm (54) das 4-Stangen-Gestänge (70) umfasst.

4. Diagnostisches Ultraschall-Bildgebungssystem nach Anspruch 3, wobei das 4-Stangen-Gestänge (70) erste und zweite Schwenkachsen (c, d) umfasst, die sich an einem Ende des zweiten oberen Arms (54) befinden, das mit dem ersten unteren Arm (52) verbunden ist, und dritte und vierte Schwenkachsen (a, b), die sich an einem Ende des zweiten oberen Arms (54) befinden, das mit der Flachbildschirmanzeige (40) verbunden ist.

5. Diagnostisches Ultraschall-Bildgebungssystem nach Anspruch 1, wobei der Verriegelungsmechanismus (92, 99) weiterhin eine vom Benutzer betätigbare Verriegelungsfreigabe (96) umfasst, die betätigt wird, um die Verriegelung der beiden Arme (52, 54) freizugeben.

6. Diagnostisches Ultraschall-Bildgebungssystem nach Anspruch 1, wobei die gelenkige Armbaugruppe (50) weiterhin eine erste vertikale Schwenkachse (122) an einem Ende des ersten unteren Arms (52) umfasst, das beweglich an dem Hauptkörper angebracht ist, und eine zweite vertikale Schwenkachse (124) an einem Ende des ersten unteren Arms (52), das mit dem zweiten oberen Arm verbunden ist.

7. Diagnostisches Ultraschall-Bildgebungssystem nach Anspruch 6, wobei die gelenkige Armbaugruppe (50) weiterhin eine dritte vertikale Schwenkachse (126) an einem Ende des zweiten oberen Arms (54) umfasst, das mit der Flachbildschirmanzeige (40) verbunden ist, und eine horizontale Schwenkachse an dem Ende des zweiten oberen Arms (54), das mit der Flachbildschirmanzeige (40) verbunden ist.

8. Diagnostisches Ultraschall-Bildgebungssystem nach Anspruch 6, wobei der Bogen des Verfahrwegs des ersten unteren Arms (52) um die erste vertikale Schwenkachse (122) so beschränkt ist, dass er weniger als 360° beträgt, und wobei der Bogen des Verfahrwegs des zweiten oberen Arms (54) um die zweite vertikale Schwenkachse 124 so beschränkt ist, dass er weniger als 360° beträgt.

9. Diagnostisches Ultraschall-Bildgebungssystem nach Anspruch 1, wobei der zweite obere Arm (54) weiterhin umfasst:
einen Pneumatikkolben (56), der wirkt, um eine Kraft zu liefern, die zumindest teilweise das Gewicht der Flachbildschirmanzeige (40) ausgleicht.

10. Diagnostisches Ultraschall-Bildgebungssystem nach Anspruch 9, weiterhin mit einem mit dem Pneumatikkolben (56) gekoppelten Justiermechanismus, der betriebsfähig ist, um die durch den Pneumatikkolben gelieferte Kraft zu justieren.

11. Diagnostisches Ultraschall-Bildgebungssystem nach Anspruch 10, wobei der Pneumatikkolben (56) justierbar ist, um eine ausgleichende Gegenkraft zu liefern, wenn der zweite obere Arm (54) in horizontaler Orientierung ausgerichtet ist.

12. Diagnostisches Ultraschall-Bildgebungssystem nach Anspruch 1, wobei der erste untere Arm (52) eine feste nach oben gerichtete Neigung von einem mit dem Hauptkörper (12) verbundenen Ende zu einem zweiten Ende aufweist, das über die Verbindung mit dem Hauptkörper angehoben ist.

13. Diagnostisches Ultraschall-Bildgebungssystem nach Anspruch 3, wobei das 4-Stangen-Gestänge (70) erste und zweite obere Stangen (72, 74), die zwischen die erste und die dritte Schwenkachse (a, c) gekoppelt sind, und dritte und vierte untere Stangen (76, 78), die zwischen die zweite und die vierte Schwenkachse (b, d) gekoppelt sind, umfasst,
wobei die erste Stange (72) starr mit der zweiten Stange (4) verbunden ist und die dritte Stange (76) starr mit der vierten Stange (78) verbunden ist.

## Revendications

1. Système d'imagerie de diagnostic par ultrasons comprenant un corps principal (12) contenant l'électronique d'imagerie et un panneau de commande (18) couplé à l'électronique d'imagerie, comprenant :
un écran plat (40) couplé électriquement à l'électronique d'imagerie ;
un ensemble de bras d'articulation (50) auquel l'écran plat (40) est raccordé pour ajuster la position de visualisation de l'écran plat, l'ensemble de bras d'articulation (50) comprenant un premier bras inférieur (52) monté de façon mobile sur le corps principal et un second bras supérieur (54) raccordé de façon mobile sur le premier bras inférieur (52) et sur l'écran plat (40), **caractérisé en ce qu'**au moins l'un des bras (52, 54) comprend une liaison à 4 barres (70) ; et
un mécanisme de verrouillage (92, 94) situé sur le premier et le second bras (52, 54) agissant pour verrouiller les deux bras (52, 54) ensemble, limitant ainsi le mouvement relatif entre les deux bras.

2. Système d'imagerie de diagnostic par ultrasons selon la revendication 1, comprenant également un chariot sur roues (14) sur lequel le corps principal (12) est monté.

3. Système d'imagerie de diagnostic par ultrasons selon la revendication 1, dans lequel le second bras supérieur (54) comprend la liaison à 4 barres (70).

4. Système d'imagerie de diagnostic par ultrasons selon la revendication 3, dans lequel la liaison à 4 barres (70) comprend un premier et un second axe d'articulation (c, d) situés à une extrémité du second bras supérieur (54) raccordée au premier bras inférieur (52), et un troisième et un quatrième axe d'articulation (a, b) situés à une extrémité du second bras supérieur (54) raccordée à l'écran plat (40).

5. Système d'imagerie de diagnostic par ultrasons selon la revendication 3, dans lequel le mécanisme de verrouillage (92, 99) comprend également un dispositif de déverrouillage utilisable par l'utilisateur (96) utilisé pour libérer le verrouillage des deux bras (52, 54).

6. Système d'imagerie de diagnostic par ultrasons selon la revendication 1, dans lequel l'ensemble de bras d'articulation (50) comprend également un premier axe d'articulation vertical (122) situé à une extrémité du premier bras inférieur (52) monté de façon mobile sur le corps principal, et un second axe d'articulation vertical (124) situé à une extrémité du premier bras inférieur (52) raccordée au second bras supérieur (54).

7. Système d'imagerie de diagnostic par ultrasons selon la revendication 6, dans lequel l'ensemble de bras d'articulation (50) comprend également un troisième axe d'articulation vertical (126) situé à une extrémité du second bras supérieur (54) raccordé à l'écran plat (40), et un axe d'articulation horizontal situé à l'extrémité du second bras supérieur (54) raccordée à l'écran plat (40).

8. Système d'imagerie de diagnostic par ultrasons selon la revendication 6, dans lequel l'arc de déplacement du premier bras inférieur (52) autour du premier axe d'articulation vertical (122) est limité pour être inférieur à 360°, et dans lequel l'arc de déplacement du second bras supérieur (54) autour du second axe d'articulation vertical (124) est limité pour être inférieur à 360°.

9. Système d'imagerie de diagnostic par ultrasons selon la revendication 1, dans lequel le second bras supérieur (54) comprend également :
un piston pneumatique (56) servant à fournir une force qui compense au moins partiellement le poids de l'écran plat (40).

10. Système d'imagerie de diagnostic par ultrasons selon la revendication 9, comprenant également un mécanisme d'ajustement, couplé au piston pneumatique (56) utilisable pour ajuster la force fournie par le piston pneumatique.

11. Système d'imagerie de diagnostic par ultrasons selon la revendication 10, dans lequel le piston pneumatique (56) est ajustable pour fournir une force contraire d'équilibrage quand le second bras supérieur (54) est orienté dans une orientation horizontale.

12. Système d'imagerie de diagnostic par ultrasons selon la revendication 1, dans lequel le premier bras inférieur (52) présente une inclinaison verticale fixe depuis une extrémité raccordée au corps principal (12) jusqu'à une seconde extrémité élevée au-dessus du raccordement au corps principal.

13. Système d'imagerie de diagnostic par ultrasons selon la revendication 3, dans lequel la liaison à 4 barres (70) comprend une première et une seconde barre supérieure (72, 74) couplées entre le premier et le troisième axe d'articulation (a, c) et la troisième et la quatrième barre inférieure (76, 78) couplées entre le second et le quatrième axe d'articulation (b, d),
dans lequel la première barre (72) est raccordée de façon rigide à la seconde barre (74) et la troisième barre (76) est raccordée de façon rigide à la quatrième barre (78).
